# EUROPEAN PATENT SPECIFICATION

(11) **EP 4 090 415 B1**
(45) Date of publication and mention of the grant of the patent: **12.06.2024**
(21) Application number: 20841696.6
(22) Date of filing: 21.12.2020
(51) Int. Cl.: A61N 1/372, A61N 1/375, A61N 1/37, A61N 1/365, A61N 1/368

(54) **LEADLESS CARDIAC PACEMAKER DEVICE CONFIGURED TO PROVIDE INTRA-CARDIAC PACING**
LEITUNGSLOSE HERZSCHRITTMACHERVORRICHTUNG MIT KONFIGURATION ZUR BEREITSTELLUNG VON INTRAKARDIALER STIMULATION
DISPOSITIF DE STIMULATEUR CARDIAQUE SANS FIL CONÇU POUR FOURNIR UNE STIMULATION INTRACARDIAQUE

(30) Priority: 13.01.2020 US 202062960172 P; 11.03.2020 EP 20162417
(43) Date of publication of application: 23.11.2022
(73) Proprietor: BIOTRONIK SE & Co. KG, 12359 Berlin (DE)
(72) Inventor: MIDGETT, Madeline Anne, Portland, OR 97213 (US); WHITTINGTON, R. Hollis, Portland, OR 97202 (US); REDDY, Ravi Kiran Kondama, Portland, OR 97221 (US); JONES, Christopher, Oregon City, OR 97045 (US); GUHANIYOGI, Shayan, Portland, OR 97210 (US)
(74) Representative: Biotronik Corporate Services SE
(86) International application number: PCT/EP2020/087361
(87) International publication number: WO 2021/144122

(56) References cited:
- US-A1- 2013 324 825
- US-A1- 2013 325 081
- US-A1- 2017 281 033
- US-B1- 9 427 594

## Description

The instant invention generally relates to a leadless cardiac pacemaker device for providing an intra-cardiac pacing, in particular a ventricular pacing, specifically VDD pacing.

In recent years, leadless pacemakers have received increasing attention. Leadless pacemakers, in contrast to pacemakers implanted subcutaneously using leads extending transvenously into the heart, avoid leads in that the pacemaker device itself is implanted into the heart, the pacemaker having the shape of a capsule for implantation into cardiac tissue, in particular the right ventricular wall of the right ventricle. Such leadless pacemakers exhibit the inherent advantage of not using leads, which can reduce risks for the patient involved with leads transvenously accessing the heart, such as the risk of pneumothorax, lead dislodgement, cardiac perforation, venous thrombosis and the like.

Leadless pacemakers may specifically be designed for implantation in the right ventricle and, in this case, during implant are placed in or on the right ventricular wall. A ventricular pacing may for example be indicated in case a dysfunction at the AV node occurs, but the sinus node function is intact and appropriate. In such a case in particular VDD pacing may be desired, involving a ventricular pacing with atrial tracking and hence requiring a sensing of atrial activity in order to pace the ventricle based on intrinsic atrial contractions.

VDD pacing is in particular motivated by patient hemodynamic benefits of atrioventricular (AV) synchrony by utilizing an appropriate sinus node function to trigger ventricular pacing, potentially allowing to maximize ventricular preload, to limit AV valve regurgitation, to maintain low mean atrial pressure, and to regulate autonomic and neurohumoral reflexes.

Whereas, to the applicant's knowledge, VDD intra-cardiac leadless pacemakers are not currently available in the market, publications have explored solutions to use modalities to detect mechanical events of atrial contractions, including the sensing of motion, sound and pressure (see for example US 2018/0021581 A1 disclosing a leadless cardiac pacemaker including a pressure sensor and/or an accelerometer to determine an atrial contraction timing). As mechanical events created by atrial contraction generally exhibit a small signal volume when sensed from the ventricle, signal based on mechanical events, for example motion, sound or pressure, may be difficult to detect. In addition, wall motion and movement of blood generated by atrial contractions may not be directly translated to the ventricle, and cardiac hemodynamic signals, such as motion, heart sounds and pressure, are likely affected by external factors such as posture and patient activity.

In document US 2013/0325081 A1 a leadless intra-cardiac medical device is described that senses cardiac activity from multiple chambers and applies cardiac stimulation to at least one cardiac chamber and/or generates a cardiac diagnostic indication. The leadless device may be implanted in a local cardiac chamber (e.g., the right ventricle) and detect near-field signals from that chamber as well as far-field signals from an adjacent chamber (e.g., the right atrium).

The invention is defined by the independent claims. It is an objective to provide a leadless pacemaker device and a method for operating a leadless pacemaker device allowing, in particular, for ventricular pacing with atrioventricular synchrony, hence requiring a reliable sensing of atrial events in order to provide for ventricular pacing based on such atrial events.

Such desires are addressed by a leadless cardiac pacemaker device configured to provide for an intra-cardiac pacing and having the features of claim 1.

In one aspect, the pacemaker device comprises a housing; an electrode arrangement arranged on the housing and configured to receive electrical signals; and a processing circuitry enclosed in the housing and operatively connected to the electrode arrangement, wherein the processing circuitry comprises a first processing channel having a first gain for processing a first processing signal derived from electrical signals received via the electrode arrangement and a second processing channel having a second gain for processing a second processing signal derived from electrical signals received via the electrode arrangement. According to an embodiment, the second gain is higher than the first gain.

The leadless pacemaker device is configured to utilize multiple processing signals. For obtaining such processing signals, an electrode arrangement is provided, the electrode arrangement comprising of one or multiple electrodes to receive electrical signals from which the processing signals are derived. The processing signals herein, for example, may be obtained each using a pair of electrodes, wherein for obtaining the different processing signals the same pair of electrodes or different pairs of electrodes may be used. In the first case, a single electrical signal, such as an intra-cardiac electrogram, may be obtained, from which different processing signals, namely the first processing signal and the second processing signal are derived for separate processing. In a second case, separate electrical signals relating for example to a ventricular sensing signal and an atrial sensing signal (i.e., by applying a sensing optimized for atrial sensing) may be received in order to derive the first processing signal and the second processing signal from such different electrical signals, the different electrical signals for example being received using the same pair of electrodes or different pairs of electrodes of the electrode arrangement. In the latter case, the separate electrical signals are for example processed in different processing channels of the processing circuitry, the different processing channels associated with different sensing circuitry. For instance, one processing channel is configured as low gain processing channel for processing the electrical signal characterizing ventricular activity, while another processing channel is configured as high gain channel for processing the electrical signal characterizing atrial activity.

The different processing signals are processed in different processing paths of the processing circuitry. For this, the processing circuitry comprises a first processing channel for processing the first processing signal, the first processing signal relating for example to a near-field (in particular ventricular) sensing signal which, according to the placement of the leadless pacemaker device for example in a ventricle of a patient's heart, may be strong such that the first processing channel may exhibit a rather low gain. In addition, the processing circuitry comprises a second processing channel for processing the second processing signal, which may relate for example to a far-field (e.g., atrial) sensing signal which, in case of a placement of the leadless pacemaker device e.g. in the ventricle, may have a small amplitude, due to the distance between the location of implantation and the source of origin of the signals. In order to allow for a reliable processing of the second processing signal, the second processing channel exhibits a gain higher than the gain of the first processing channel, such that features relating to an atrial activity may be suitably analyzed within the received signals.

Because, for a placement of the leadless pacemaker device in for example the ventricle, atrial activity occurs in the far-field, atrial events within a regular ventricular sensing signal (for example obtained via a regular ventricular QRS sensing channel) may be hard to discern, as a P wave stemming from atrial activity may exhibit a small amplitude in relation to QRS and T waves. For this reason signal portions relating to far-field activity may be processed separately from signals relating to near-field activity within the second processing channel, such that within the second processing channel far-field events may be detected with increased reliability and enhanced timing precision.

The housing provides for a hermetic sealing of the leadless pacemaker device, the leadless pacemaker device including all required components for autarkic operation, such as the processing circuitry, an energy storage such as a battery, electric and electronic circuitry and the like, within the housing. The housing is fluid-tight such that the leadless pacemaker device may be implanted into cardiac tissue and may be kept in cardiac tissue over an extended period of time to provide continuous cardiac pacing operation.

The leadless pacemaker device, in one aspect, is to be placed in the right or left ventricle.

In one aspect, the leadless pacemaker device comprises a fixation device having at least one fixation element arranged at the tip of the housing for fixing the pacemaker device to intra-cardiac tissue, in particular the ventricular wall. In one embodiment, one or multiple fixation elements in the shape of thin wires, for example Nitinol tines exhibiting a shape memory effect, may be provided. In another embodiment, a fixation element in the shape of a screw anchor may be provided.

In one aspect, the electrode arrangement comprises a first electrode arranged in the vicinity of a tip of the housing. The first electrode shall come to rest on cardiac tissue in an implanted state of the pacemaker device, such that the first electrode contacts cardiac tissue at a location effective for injecting a stimulating signal into cardiac tissue for provoking a pacing action, in particular a ventricular pacing.

In one aspect, the electrode arrangement comprises a second electrode formed by an electrode ring circumferentially extending about the housing. Alternatively, the second electrode may for example be formed by a patch or another electrically conductive area formed on the housing. The second electrode is placed at a distance from the tip of the housing and hence at a distance from the first electrode arranged at the tip.

In one embodiment, the processing circuitry is configured to process, as said first processing signal, a first signal sensed between the first electrode and the second electrode. Such first signal may be denoted as near-field vector to be received between a pair of electrodes comprised of the first electrode and the second electrode. As the first electrode and the second electrode may, in one embodiment, be located at a rather close distance to each other, such pair of electrodes is predominantly suited to receive signals in close proximity to the leadless pacemaker device, i.e., in the near-field region within the ventricle if the leadless pacemaker device is implanted into the ventricle. The sense signal received in between the first electrode and the second electrode is provided to the first processing channel for processing in order to for example detect near-field (e.g., ventricular) events in the signal.

In one embodiment, the housing comprises a far end opposite the tip, the electrode arrangement comprising a third electrode arranged on the housing at the far end opposite the tip. The third electrode is operatively connected to the processing circuitry, such that the processing circuitry is enabled to receive and process signals received via the third electrode.

In one aspect, the processing circuitry is configured to process, as said second processing signal, a second signal sensed between the first electrode and the second or the third electrode. Such second signal vector arising between the first electrode and the second or the third electrode may be referred to as far-field vector, the first electrode and the third electrode exhibiting a distance with respect to each other larger than the first and the second electrode. The second signal may in particular be processed to detect events in the far-field, i.e., atrial contractions in case the leadless pacemaker device is placed in the ventricle, such that by means of the second signal an intrinsic atrial activity prior to injecting a pacing stimulus may be captured.

The second signal sensed between the first electrode and the third electrode may be used to sense intrinsic atrial contractions in order to provide for an atrial to ventricular synchronization by timely injecting a stimulus at the ventricular location of implantation of the pacemaker device following atrial contractions. The second signal is provided to the second processing channel in order to process the signal and detect atrial events from the signal, in order to provide for a pacing action based on detected atrial events, hence allowing for a ventricular pacing under atrioventricular (AV) synchrony.

In one aspect, the same set (or sub-set) of electrodes of the leadless pacemaker device is used both for sensing contraction signals as well as for emitting pacing stimulation signals. For this, in one embodiment, the processing circuitry of the leadless pacemaker device is configured to switch between a sensing mode and a stimulus mode by alternating between the processing of received signals and the generation of pacing signals. In particular, the processing circuitry may be configured to, in a first phase of the cardiac cycle, sense for atrial contractions. If atrial contractions are detected, the processing circuitry may sense ventricular activity within a time delay after the sensed atrial event. If no ventricular activity is sensed, the pacemaker device may switch to a stimulus mode in which a pacing signal is generated and emitted using at least one of the first electrode and the second electrode. After the pacing signal has been emitted, atrial contractions may be sensed anew to continue pacing operation.

In one embodiment, the second processing channel comprises a processing stage for differentiating one wave portion from another wave portion in the second processing signal. The processing stage in particular may be configured to apply, to the second processing signal, may include bandpass filtering, a blanking window for excluding a portion of the second reception signal from further processing, a moving average filtering, and a rectification. By means of the processing stage, in particular such wave portions shall be isolated and/or emphasized within the signal to be processed which may be indicative of e.g. an atrial event. If the leadless pacemaker device is placed in the ventricle of a patient's heart, signal portions relating to far-field (e.g. atrial) activity may have a much smaller amplitude than signal portions relating to a near-field (e.g. ventricular) activity. Hence, the processing serves to differentiate between the different signal portions in order to identify such signal portion which may contain signals relating to far-field (atrial) activity, namely the so-called P wave.

For isolating the P wave, a bandpass filtering may be applied, hence differentiating wave portions relating to the P wave from wave portions in particular relating to QRS and T waves stemming from ventricular activity. Alternatively or in addition, a blanking method may be applied in order to blank out certain portions of the second processing signal, namely such portions which contain signals stemming from events other than a far-field (e.g.) atrial activity. A blanking window, for this, serves to silence signal portions which are not of interest for far-field activity, but which may rather interfere with the detection of far-field activity. By means of a blanking window such portions of the signal which do not relate to far-field (e.g. atrial) activity hence are excluded from processing, such that the processing is limited to those signal portions (likely) relating to far-field activity. Alternatively or in addition, other methods such as a moving average filtering, finite differences or a rectification of the signal may be applied. A moving averaging filter herein can be used to smooth the processing signal. Rectification can serve to easily compare the processed signal to a (single) threshold in order to identify when the signal magnitude exceeds a predefined threshold. The rectified processed signal can also be compared to an adaptable threshold.

In one embodiment, the first processing channel comprises a first detection stage for detecting at least one near-field event in the first reception signal. Herein, the processing stage of the second processing channel may be configured to determine at least one limit of a blanking window for excluding a portion of the second reception signal from further processing based on a near-field (e.g. ventricular) event detected by the first detection stage of the first processing channel. The first processing channel serves to process a processing signal at a lower gain for detecting near-field events, i.e., events that are due to an activity in close proximity to the implanted leadless pacemaker device, for example in the ventricle in which the leadless pacemaker device is implanted. As the near-field event will also be picked up in the second processing signal, it is advantageous to blank out such a signal portions relating to a near-field activity, i.e., QRS and T waves in in case of a placement of the leadless pacemaker device in the ventricle. In order to correctly locate the blanking window, detected near-field events may be taken into account, in order to for example determine a regular timing in between atrial events and ventricular events. From detected near-field events it may be determined in what time range after a far-field event a near-field event typically should occur, such that the blanking window, defined by a start time and a stop at time, may be appropriately set to blank out such portions of the second processing signal which relates to the near-field (ventricular) events.

During the blanking window the second processing channel may, at least partially, be switched off in order to for example save power within the second, high gain processing channel. The second processing channel may for example comprise an amplification stage for amplifying the second processing signal, wherein the amplification stage may be switched off during the period of the blanking window such that that no power is consumed by amplification during the time interval of the blanking window.

A detection of far-field (atrial) events takes place in a detection window outside a blanking window. The detection window herein may start (immediately) at the end of a prior blanking window and may end at the beginning of the next blanking window. It however is also conceivable that the detection window for example starts at some time delay after the end of a prior blanking window. In between the end of the blanking window and the start of the detection window the second processing channel may be fully functional and process the associated second processing signal, wherein however a detection of far-field (atrial) events does not take place until the start of the detection window.

In one embodiment, the second processing channel comprises a second detection stage for detecting at least one far-field event in the second processing signal. The second detection stage may be arranged logically behind the processing stage of the second processing channel, such that the second detection stage receives processed signals from the processing stage of the second processing channel. The second detection stage herein serves to identify far-field (atrial) events in the second processing signal in order to output information relating to the timing of a detected far-field event.

The second detection stage of the second processing channel in particular may be configured to detect an atrial event by comparing the second processing signal to a threshold. In case the magnitude of the second processing signal exceeds a threshold, it may be concluded that a far-field event is present. The processing herein may take place on a rectified signal, which makes it possible to apply a single threshold to which the rectified signal may be compared. It however is also possible to identify a far-field (atrial) event in a non-rectified signal, for example by applying two thresholds, namely a positive threshold and a negative threshold, wherein a far-field (atrial) event is identified if the positive signal portion exceeds the positive threshold and/or the (magnitude of the) negative signal portion exceeds the negative threshold.

The threshold(s) herein may be adaptable. For example, the threshold(s) may be set based on a maximum and/or minimum of a detected P wave. The threshold(s) may for example be set according to a predefined percentage of a maximum or minimum of a prior detected P wave.

Alternatively or in addition, one or multiple morphological features of the second processing signal may be evaluated in order to confirm or identify a far-field (atrial) event. The evaluation of one or multiple morphological features herein may include the determining of an amplitude value, the determining of a duration of a wave portion, for example of a P wave, the determining of a number of threshold crossings or zero crossings, and/or the determining of a location of a wave portion in a detection window.

Generally, the identification of a far-field (atrial) event by comparing the second processing signal to one or multiple thresholds within the second processing channel allows for a fast detection and identification of a far-field (atrial) event. However, such detection possibly may not be fully accurate, due to for example a limited signal-to-noise ratio of far-field signal portions. In order to, hence, allow for a fast, yet accurate detection and identification of atrial events, it may be beneficial to confirm far-field (atrial) events detected by comparison to a threshold by evaluating morphological features of the processing signal processed in the second processing channel. This allows to for example confirm true P waves and to reject false detections stemming from noise, QRS waves or T waves. As the evaluation of morphological features, for example by determining the length of a wave form, potentially requires an evaluation within a certain time interval, the confirmation may come after some delay from an initial identification of a far-field (atrial) event detected by a threshold crossing.

Hence, a far-field (atrial) may be preliminary detected by a threshold crossing, causing for example an atrial sense marker to be output by the second processing channel, the atrial sense marker being canceled later if the evaluation according to morphological features does not confirm the atrial event. Alternatively, an atrial sense marker may be output by the second processing channel only after confirmation according to morphological features, at the expense however of a potential delay of the output of the atrial sense marker with respect to the actual occurrence of the P wave.

In one aspect, the processing circuitry is configured to trigger a pacing signal based on at least one detected far-field (e.g., atrial) event. The leadless pacemaker device may provide for a synchronous pacing. For this, if no intrinsic ventricular activity is detected via the first processing channel within a defined delay time window, a simulation signal may be emitted by the pacemaker device for stimulating ventricular activity.

In one embodiment, in the event of loss of far-field (atrial) tracking due to for example a non-discernible contraction feature in the second processing signal, far-field tracking could temporarily not be based on the electrical signal received by means of the electrode arrangement, until a far-field tracking feature in the second processing signal is again identified. A pacing action (in particular for a ventricular pacing) could in this case be timed using an alternate atrial sensor, such as a motion sensor, impedance signal, or another indicator of atrial activity. Alternatively, the pacing could be switched to an asynchronous pacing mode. When a far-field activity cannot be tracked, the second processing channel may process the second processing signal in order to monitor continuously or at periodic intervals to determine when far-field events return in a possibly adaptive detection window.

The various features and advantages of the present invention may be more readily understood with reference to the following detailed description and the embodiments shown in the drawings. Herein,
- Fig. 1: shows a schematic view of the human heart;
- Fig. 2: shows a schematic view of a leadless pacemaker device;
- Fig. 3: shows a schematic view of a leadless pacemaker device, indicating signal vectors between different electrodes of the leadless pacemaker device;
- Fig. 4: shows a schematic view of a processing circuitry of an embodiment of a leadless pacemaker device;
- Fig. 5A: shows a first processing signal in the shape of an intra-cardiac electrogram (IEGM) received by a first processing channel of the processing circuitry;
- Fig. 5B: shows a second processing signal, in a raw state prior to processing by a second processing channel;
- Fig. 5C: shows the second processing signal, after processing by means of a processing stage of the second processing channel; and
- Fig. 5D: shows the timing of detected ventricular and atrial events in the first processing signal and the second processing signal.

Subsequently, embodiments of the invention shall be described in detail with reference to the drawings. In the drawings, like reference numerals designate like structural elements.

It is to be noted that the embodiments are not limiting for the invention, but merely represent illustrative examples.

In the instant invention it is proposed to provide a leadless pacemaker device providing for an intra-cardiac pacing, in particular a ventricular pacing, specifically a so-called VDD pacing.

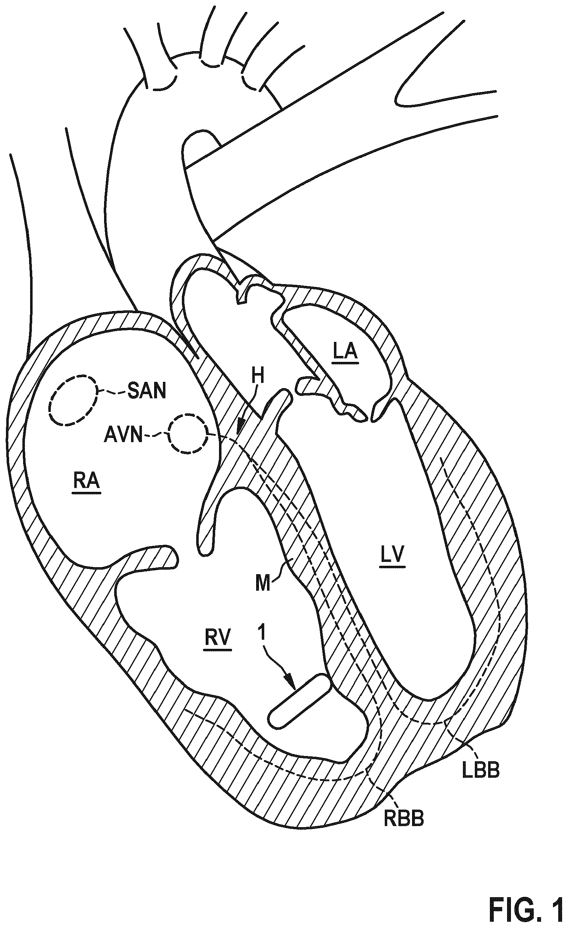

Fig. 1 shows, in a schematic drawing, the human heart comprising the right atrium RA, the right ventricle RV, the left atrium LA and the left ventricle LV, the so-called sinoatrial node SAN being located in the wall of the right atrium RA, the sinoatrial node SAN being formed by a group of cells having the ability to spontaneously produce an electrical impulse that travels through the heart's electrical conduction system, thus causing the heart to contract in order to pump blood through the heart. The atrioventricular node AVN serves to coordinate electrical conduction in between the atria and the ventricles and is located at the lower back section of the intra-atrial septum near the opening of the coronary sinus. From the atrioventricular node AVN the so-called HIS bundle H is extending, the HIS bundle H being comprised of heart muscle cells specialized for electrical conduction and forming part of the electrical conduction system for transmitting electrical impulses from the atrioventricular node AVN via the so-called right bundle branch RBB around the right ventricle RV and via the left bundle branch LBB around the left ventricle LV.

In case of a block at the atrioventricular node AVN, the intrinsic electrical conduction system of the heart H may be disrupted, causing a potentially insufficient intrinsic stimulation of ventricular activity, i.e., insufficient or irregular contractions of the right and/or left ventricle RV, LV. In such a case, a pacing of ventricular activity by means of a pacemaker device may be indicated, such pacemaker device stimulating ventricular activity by injecting stimulation energy into intra-cardiac tissue, specifically myocardium M.

Within the instant text, it is proposed to use a leadless cardiac pacemaker device 1, as schematically indicated in Fig. 1, for providing for a ventricular pacing action.

Whereas common leadless pacemaker devices are designed to sense a ventricular activity by receiving electrical signals from the ventricle RV, LV they are placed in, it may be desirable to provide for a pacing action which achieves atrioventricular (AV) synchrony by providing a pacing in the ventricle in synchrony with an intrinsic atrial activity. For such pacing mode, also denoted as VDD pacing mode, it is required to sense atrial activity and identify atrial events relating to atrial contractions in order to base a ventricular pacing on such atrial events.

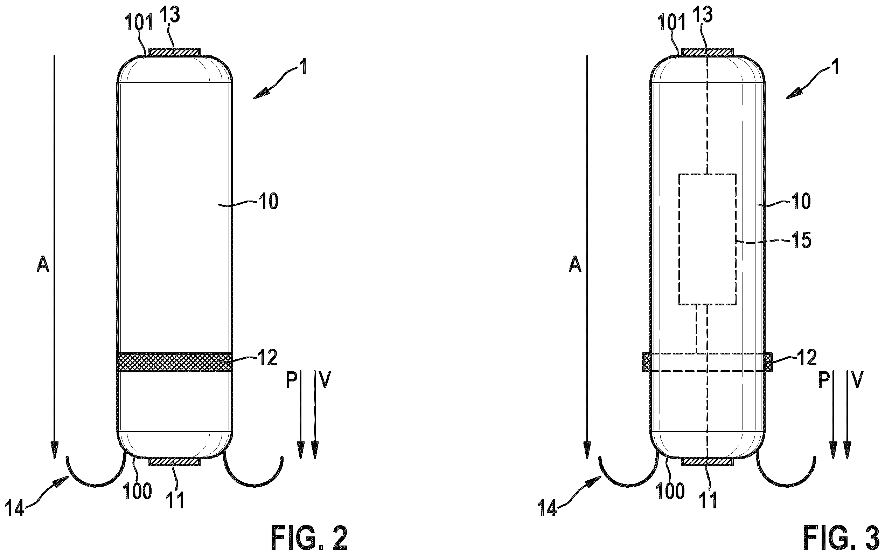

Referring now to Figs. 2 and 3, in one embodiment a leadless pacemaker device 1 configured to provide for an intra-cardiac pacing, in particular in a VDD pacing mode, comprises a housing 10 enclosing electrical and electronic components for operating the leadless pacemaker device 1. In particular, enclosed within the housing 10 is a processing circuitry 15, comprising for example also a communication interface for communicating with an external device, such as a programmer wand. In addition, electrical and electronic components such as an energy storage in the shape of a battery are confined in the housing 10. The housing 10 encloses components received therein, the housing 10 having the shape of, e.g., a cylindrical shaft having a length of for example a few centimeters.

The leadless pacemaker device 1 is to be implanted immediately on intra-cardiac tissue M. For this, the leadless pacemaker device 1 comprises, in the region of the tip 100, a fixation device 14 for example in the shape of nitinol wires to engage with intra-cardiac tissue M for fixedly holding the leadless pacemaker device 1 on the tissue in an implanted state.

The leadless pacemaker device 1 does not comprise leads, but receives signals relating to a cardiac activity by means of an electrode arrangement arranged on the housing 10 and also emits stimulation signals by means of such electrode arrangement. In the embodiment of Figs. 2 and 3, the leadless pacemaker device 1 comprises different electrodes 11, 12, 13 making up the electrode arrangement and serving to emit pacing signals towards intra-cardiac tissue M for providing a pacing and to sense electrical signals indicative of a cardiac activity, in particular indicative of atrial and ventricular contractions.

A first electrode 11 herein is denoted as pacing electrode. The first electrode 11 is placed at a tip 100 of the housing 10 and is configured to engage with cardiac tissue M.

A second electrode 12 serves as a counter-electrode for the first electrode 11, a signal vector P arising between the first electrode 11 and the second electrode 12 providing for a pacing vector P for emitting pacing signals towards the intra-cardiac tissue M.

In addition, the second electrode 12 serves as a sensing electrode for sensing signals, in particular relating to ventricular contractions, a signal vector V arising between the second electrode 12 and the first electrode 11, the signal vector V being denoted as near-field vector.

The second electrode 12 is placed at a distance from the first electrode 11 and for example has the shape of a ring extending circumferentially about the housing 10. The second electrode 12 is for example placed at a distance of about 1 cm from the tip 100 of the housing 10 at which the first electrode 11 is placed.

The leadless pacemaker device 1, in the embodiment of Figs. 2 and 3, in addition comprises a third electrode 13 placed at a far end 101 of the housing 10, the third electrode 13 serving as a sensing electrode for sensing signals indicative of cardiac activity in the far-field. In particular, a signal vector A arises between the third electrode 13 and the first electrode 11, the signal vector A picking up signals being indicative for example of atrial contractions and being denoted as far-field vector.

The electrodes 11, 12, 13 are in operative connection with the processing circuitry 15, the processing circuitry 15 being configured to cause the first electrode 11 and the second electrode 12 to emit a pacing signal for providing a stimulation at the ventricle. The processing circuitry 15 furthermore is configured to process signals received via the electrodes 11, 12, 13 to provide for a sensing of cardiac activity, in particular atrial and ventricular contractions.

In order to provide for a pacing in the ventricle in which the leadless pacemaker device 1 is placed, in particular to enable a pacing in the VDD mode, a sensing of atrial activity is required to provide for detected atrial sense markers in order to time a pacing in the ventricle to obtain atrioventricular (AV) synchrony. For this, a far-field signal from in particular the right atrium RA (see Fig. 1) shall be sensed in order to allow for a synchronous pacing in the right ventricle RV by means of the leadless pacemaker device 1 being implanted on intra-cardiac tissue M in the right ventricle RV.

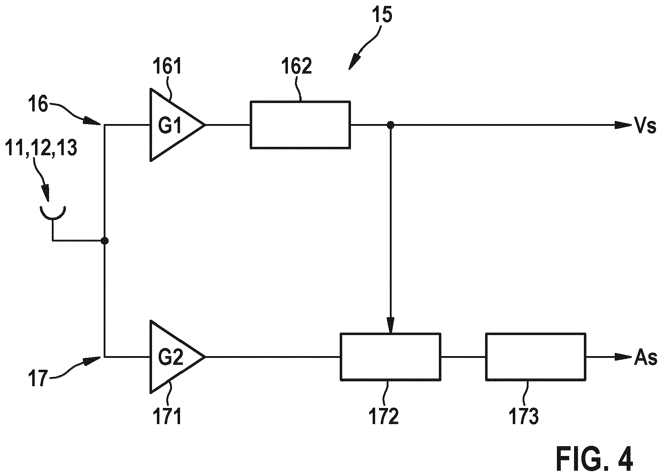

Referring now to Fig. 4, the processing circuitry 15 comprises, in one embodiment, two processing channels 16, 17 for processing different processing signals relating to ventricular activity and atrial activity. Herein, typically, an intra-cardiac electrogram (IEGM) contains a signal portions relating to ventricular activity (in particular a QRS wave) and atrial activity (in particular a P wave), signal portions relating to atrial activity however resulting from a far-field signal source and hence being far less pronounced and having a far smaller amplitude then signal portions relating to a ventricular activity in the near-field, i.e., arising in close proximity to the implanted leadless pacemaker device 1. For this reason, the two processing channels 16, 17 are associated with different gains G1, G2, a first processing channel 16 serving to process a first processing signal to identify ventricular events at a rather low gain G1 and a second processing channel 17 being configured to process a second processing signal to identify atrial events at a significantly higher gain G2.

In particular, the first processing channel 16 is connected to the electrode arrangement comprised of the electrodes 11, 12, 13, the first processing channel 16 being configured in particular to sense and process a signal received via the electrodes 11, 12 (near-field vector V in Figs. 2 and 3). The first processing channel 16 comprises a first amplification stage 161 having a gain G1 and, following the amplification stage 161, a detection stage 162 which is configured to identify ventricular sense markers Vs from the first processing signal processed within the first processing channel 16.

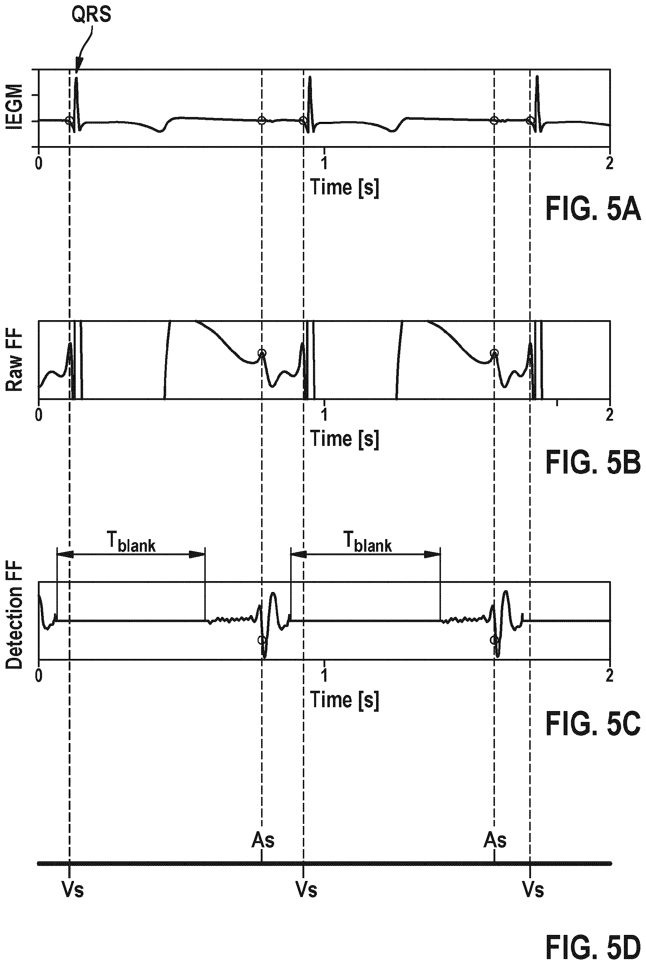

This is illustrated in Fig. 5A. The first processing signal processed within the first processing channel 16 resembles an intra-cardiac electrogram (IEGM) picked up in the ventricle in which the leadless pacemaker device 1 is implanted, the signal including portions relating to ventricular activity as well as atrial activity, signal portions relating to a ventricular activity however being far pronounced with respect to other signal portions (QRS waves as visible from Fig. 5A). For example by means of comparing the signal to a suitable threshold or by applying a morphological analysis, the occurrence of ventricular events, in particular the start of a QRS wave, may be discerned, the second processing channel 16 outputting ventricular sense markers Vs indicative of such ventricular events.

The second processing channel 17 is likewise connected to the electrode arrangement comprised of electrodes 11, 12, 13, wherein the second processing channel 17 may in particular be configured to process a signal sensed via the far-field vector A, that is in between the electrodes 11, 13 placed at the tip 100 and the far end 101 of the housing 10 as illustrated in Figs. 2 and 3. The second processing channel 17 comprises a second amplification stage 171 having a second gain G2, the second amplification stage 171 being followed by a processing stage 172 and a second detection stage 173.

The processing stage 172 serves to pre-process the second processing signal after amplification. The detection stage 173 in turn serves to evaluate and analyze the processed signal in order to identify atrial events within the second processing signal, the second processing channel 17 then outputting atrial sense markers As indicative of atrial events detected in the processed signal.

In order to identify and analyze atrial events, the gain G2 of the second processing channel 17 could be (significantly) higher than the gain G1 of the first processing channel 16. As visible from Fig. 5B illustrating the second processing signal in a raw state prior to processing by means of the processing stage 172, this allows to analyze signal portions relating to atrial events, but makes it necessary to discern such signal portions relating to atrial events from other signal portions, in particular signal portions relating to ventricular events in the near-field and hence being far stronger than signal portions originating from atrial events in the far-field.

Within the processing stage 172, for example a bandpass filtering, a partial blanking, a smoothing by means of a moving average filtering and a rectification may take place. A first or second order difference may be applied to remove a non-zero baseline while enhancing P wave detections.

Fig. 5C illustrates the signal after processing by means of the processing stage 172 in the second processing channel 17. In particular, within the processing a blanking window T_{blank} may be applied serving to mute the signal in such time intervals which - likely - do not relate to an atrial activity. The position of the blanking window T_{blank} herein is determined, in the embodiment of Fig. 4, according to an identification of prior ventricular events by means of the first processing channel 16.

In particular, by means of the detection of ventricular events in the first processing channel 16 a timing in between atrial events and ventricular events may be determined. According to such timing a start point and an end point of the blanking window T_{blank} may be set, hence excluding signal portions from the processing which do not relate to atrial activity. Strong ventricular signals in this way may be suppressed such that signal portions relating to a ventricular activity may not interfere with a detection of atrial events.

During the blanking window T_{blank}, the second processing channel 17 may be turned off. In particular, the amplification stage 171 of the second processing channel 17 may be switched of in order to save power.

Generally, a detection for atrial events takes place outside of the blanking window T_{blank}. Herein, a detection window for detecting atrial events may start at the end of a prior blanking window T_{blank}. Alternatively, a detection window may have a delay with respect to the end of a prior blanking window T_{blank}, such that a signal processing within the second processing channel 17 starts at the end of a prior blanking window T_{blank}, a detection for atrial events however starting only after a certain delay.

For detecting atrial events within the second processing signal processed in the second processing channel 17 in order to output atrial sense markers As illustrated in Fig. 5D, different measures may be taken.

In order to allow for a fast detection of atrial events, the signal may for example be rectified within the processing stage 172, the detection stage 173 being configured to monitor whether the processed signal exceeds a (positive) threshold. Alternatively, no rectification may be applied in the processing stage 172, the detection stage 173 monitoring for a crossing of a positive threshold or a negative threshold (or both).

Such thresholds herein may be continuously adapted according to a detected P wave amplitude. In particular, the thresholds for a following cycle for a subsequent atrial event detection may be set according to a predefined percentage of the amplitude of a detected P wave in a prior cycle.

By monitoring a threshold crossing, a fast detection of atrial events is possible, however at the expense of a potentially reduced reliability. Therefore, in one embodiment, a further analysis of for example morphological features of the signals, in particular a detected P wave, may be applied in order to confirm an atrial event or, in the negative, to reject a false detection of an atrial event. For such morphological analysis, for example the amplitude of a detected P wave, the duration of a detected P wave, a number of threshold crossings or zero crossings in a detected P wave, and/or a location of a detected P wave in the detection window may be evaluated. In case it is concluded from the morphological analysis that a true atrial event is present, the atrial event identified by the initial threshold crossing monitoring is confirmed. Otherwise, the atrial event is rejected as false.

As the morphological analysis requires an evaluation of the signal within a certain time interval, confirmation or rejection by means of the morphological analysis may come at a delay with respect to the identification according to a threshold crossing. It herein is possible to output an atrial sense marker. As already at the initial identification by means of a threshold crossing in order to then, after the morphological analysis, cancel the atrial sense markers in case of a false detection. Alternatively, an atrial sense marker As may only be output after a confirmation by means of the morphological analysis, at the expense of an increased delay in between a true occurrence of a P wave and the output of the atrial sense marker As.

Using atrial sense markers As output by the second processing channel 17, a ventricular synchronous pacing may be achieved. For this, it can be detected whether, following a detected atrial sense marker As, an intrinsic ventricular sense marker Vs occurs (output by the first processing channel 16) within a predefined time delay window after the atrial sense marker As, in which case no stimulation is required. If no ventricular sense marker Vs is detected, a stimulation pulse may be emitted, causing a synchronous pacing at the ventricle.

Conversely, also an asynchronous pacing can be performed.

Utilizing a far-field electrical signal received by means of a leadless pacemaker device can offer a superior detection of far-field events, in particular atrial events in case the leadless pacemaker device is implanted into the ventricle. A tracking of far-field events by using and evaluating electrical signals may allow for an increased consistency and reliability in particular with respect to external factors such as posture and patient activity.

### List of reference numerals

- 1: Leadless pacemaker device
- 10: Housing
- 100: Tip
- 101: Far end
- 11: First electrode (pacing electrode)
- 12: Second electrode (pacing ring)
- 13: Third electrode
- 14: Fixation device
- 15: Processing circuitry
- 16: Processing channel
- 160: Electrode arrangement
- 161: Amplification stage
- 162: Detection stage
- 17: Processing channel
- 170: Electrode arrangement
- 171: Amplification stage
- 172: Processing stage
- 173: Detection stage
- A: Atrial vector
- As: Atrial event
- AVN: Atrioventricular node
- G1, G2: Gain
- H: HIS bundle
- LA: Left atrium
- LBB: Left bundle branch
- LV: Left ventricle
- M: Intra-cardiac tissue (myocardium)
- P: Pacing vector
- RA: Right atrium
- RBB: Right bundle branch
- RV: Right ventricle
- SAN: Sinoatrial node
- T_{blank}: Blanking window
- V: Ventricular vector
- Vs: Ventricular event

## Claims

1. A leadless pacemaker device (1) configured to provide for an intra-cardiac pacing, the leadless pacemaker device (1) comprising:
a housing (10);
an electrode arrangement arranged on the housing (10) and configured to receive electrical signals; and
a processing circuitry (15) enclosed in the housing (10) and operatively connected to the electrode arrangement, wherein the processing circuitry (15) comprises a first processing channel (16) having a first gain (G1) for processing a first processing signal derived from electrical signals received via the electrode arrangement and a second processing channel (17) having a second gain (G2) for processing a second processing signal derived from electrical signals received via the electrode arrangement, the second gain (G2) being higher than the first gain (G1), wherein
the second processing channel (17) comprises a processing stage (172) that is configured to differentiate one wave portion from another wave portion in the second processing signal and apply, to the second processing signal, a blanking window for excluding
a portion of the second processing signal from further processing; and wherein
the second processing channel (17) comprises an amplification stage (170) for amplifying the second processing signal, wherein the amplification stage (170) is switched off during said blanking window (Tblank).

2. The leadless pacemaker device (1) according to claim 1, wherein the housing (10) comprises a tip (100), the electrode arrangement comprising a first electrode (11) placed on the tip (100) for engaging with intra-cardiac tissue (M).

3. The leadless pacemaker device (1) according to claim 2, wherein the electrode arrangement comprises a second electrode (12) formed by an electrode ring circumferentially extending about the housing (10) and being placed at a distance from said tip (100).

4. The leadless pacemaker device (1) according to claim 3, wherein the processing circuitry (15) is configured to process, as said first and/or second processing signal, a first and/or second signal sensed between the first electrode (11) and the second electrode (12).

5. The leadless pacemaker device (1) according to at least one of claims 2 to 4, wherein the housing (10) comprises a far end (101) opposite the tip (100), the electrode arrangement comprising a third electrode (14) arranged in the vicinity of the far end (101).

6. The leadless pacemaker device (1) according to claim 5, wherein the processing circuitry (15) is configured to process, as said first and/or second processing signal, a first and/or second signal sensed between the first electrode (11) and the third electrode (13).

7. The leadless pacemaker device (1) according to one of the preceding claims, wherein the processing circuitry (15) is configured to process the first processing signal to detect a ventricular activity and the second processing signal to detect an atrial activity.

8. The leadless pacemaker device (1) according to one of the preceding claims, wherein the first processing channel (16) comprises a first detection stage (162) for detecting at least one near-field event (Vs) in the first processing signal, wherein the processing stage (172) of the second processing channel (17) is configured to determine at least one limit of a blanking window (T_{blank}) for excluding a portion of the second processing signal from further processing based on at least one near-field event (Vs) detected by said first detection stage (162).

9. The leadless pacemaker device (1) according one of the preceding claims, wherein the second processing channel (17) comprises a second detection stage (173) for detecting at least one far-field event (As) in the second processing signal.

10. The leadless pacemaker device (1) according to claim 9, wherein the second detection stage (173) is configured to detect said at least one far-field event (As) by at least one of
- comparing the second processing signal to a threshold, and evaluating at least one morphological feature of the second processing signal, wherein the evaluating of at least one morphological feature includes at least one of determining an amplitude value from the second processing signal,
- determining a duration of a wave portion of the second processing signal,
- determining a number of threshold crossings or zero crossings in the second processing signal, and
- determining a location of a wave portion in a detection window.

11. The leadless pacemaker device (1) according to claim 9 or 10, wherein the processing circuitry (15) is configured to trigger a pacing signal based on at least one detected far-field event (As).

12. Method for operating a leadless pacemaker device (1) for providing intra-cardiac pacing, comprising:
receiving electrical signals, using an electrode arrangement arranged on a housing (10) of the leadless pacemaker device (1); and
processing, using a processing circuitry (15) enclosed in the housing (10) and operatively connected to the electrode arrangement, a first processing signal and a second processing signal derived from electrical signals received via the electrode arrangement, wherein the processing circuitry (15) comprises a first processing channel (16) having a first gain (G1) for processing said first processing signal and a second processing channel (17) having a second gain (G2) for processing said second processing signal, the second gain (G2) being higher than the first gain (G1);
differentiating by a processing stage (172) that is comprised by the second processing channel (17) one wave portion from another wave portion in the second processing signal; applying by the processing stage (172) that is comprised by the second processing channel (17), to the second processing signal, a blanking window for excluding a portion of the second processing signal from further processing; and
amplifying, by an amplification stage (170) that is comprised by the second processing channel (17), the second processing signal, wherein the amplification stage (170) is switched off during said blanking window (Tblank).

## Patentansprüche

1. Kabelloses Herzschrittmachergerät (1), dafür konfiguriert, eine intrakardiale Stimulation bereitzustellen, wobei das Herzschrittmachergerät (1) umfasst:
ein Gehäuse (10);
eine Elektrodenanordnung, die am Gehäuse (10) angeordnet und dafür konfiguriert ist, elektrische Signale zu empfangen; und
eine Verarbeitungsschaltung (15), die im Gehäuse (10) eingeschlossen und mit der Elektrodenanordnung wirkverbunden ist, wobei die Verarbeitungsschaltung (15) einen ersten Verarbeitungskanal (16) mit einer ersten Eingangsverstärkung (G1) zum Verarbeiten eines ersten Verarbeitungssignals, das aus elektrischen Signalen abgeleitet ist, die über die Elektrodenanordnung empfangen werden, und einen zweiten Verarbeitungskanal (17) mit einer zweiten Eingangsverstärkung (G2) zum Verarbeiten eines zweiten Verarbeitungssignals, das aus elektrischen Signalen abgeleitet ist, die über die Elektrodenanordnung empfangen werden, umfasst, wobei die zweite Eingangsverstärkung (G2) höher als die erste Eingangsverstärkung (G1) ist, wobei
der zweite Verarbeitungskanal (17) eine Verarbeitungsstufe (172) umfasst, die dafür konfiguriert ist, einen Wellenabschnitt von einem anderen Wellenabschnitt im zweiten Verarbeitungssignal zu unterscheiden, und
auf das zweite Verarbeitungssignal ein Ausblendungsfenster anzuwenden, um einen Abschnitt des zweiten Verarbeitungssignals von der weiteren Verarbeitung auszuschließen, und wobei der zweite Verarbeitungskanal (17) eine Verstärkungsstufe (170) umfasst, um das zweite Verarbeitungssignal zu verstärken, wobei die Verstärkungsstufe (170) während besagtem Ausblendungsfenster (T_{blank}) abgeschaltet ist.

2. Kabelloses Herzschrittmachergerät (1) nach Anspruch 1, wobei das Gehäuse (10) eine Spitze (100) umfasst, wobei die Elektrodenanordnung eine erste Elektrode (11) umfasst, die auf der Spitze (100) platziert ist, um in intrakardiales Gewebe (M) einzugreifen.

3. Kabelloses Herzschrittmachergerät (1) nach Anspruch 2, wobei die Elektrodenanordnung eine zweite Elektrode (12) umfasst, die durch einen Elektrodenring ausgebildet ist, der sich umlaufend um das Gehäuse (10) erstreckt und in einem Abstand zu besagter Spitze (100) platziert ist.

4. Kabelloses Herzschrittmachergerät (1) nach Anspruch 3, wobei die Verarbeitungsschaltung (15) dafür konfiguriert ist, als besagtes erstes und/oder zweites Verarbeitungssignal, ein erstes und/oder zweites Signal zu verarbeiten, das zwischen der ersten Elektrode (11) und der zweiten Elektrode (12) erfasst wird.

5. Kabelloses Herzschrittmachergerät (1) nach mindestens einem der Ansprüche 2 bis 4, wobei das Gehäuse (10) ein entferntes Ende (101) der Spitze (100) gegenüberliegend umfasst, wobei die Elektrodenanordnung eine dritte Elektrode (14) umfasst, die in der Nähe des entfernten Endes (101) angeordnet ist.

6. Kabelloses Herzschrittmachergerät (1) nach Anspruch 5, wobei die Verarbeitungsschaltung (15) dafür konfiguriert ist, als besagtes erstes und/oder zweites Verarbeitungssignal, ein erstes und/oder zweites Signal zu verarbeiten, das zwischen der ersten Elektrode (11) und der dritten Elektrode (13) erfasst wird.

7. Kabelloses Herzschrittmachergerät (1) nach einem der vorstehenden Ansprüche, wobei die Verarbeitungsschaltung (15) dafür konfiguriert ist, das erste Verarbeitungssignal zu verarbeiten, um eine ventrikuläre Aktivität zu detektieren, und das zweite Verarbeitungssignal, um eine atriale Aktivität zu erkennen.

8. Kabelloses Herzschrittmachergerät (1) nach einem der vorstehenden Ansprüche, wobei der erste Verarbeitungskanal (16) eine erste Detektionsstufe (162) zum Detektieren mindestens eines Nahfeldereignisses (Vs) im ersten Verarbeitungssignal umfasst, wobei die Verarbeitungsstufe (172) des zweiten Verarbeitungskanals (17) dafür konfiguriert ist, mindestens eine Grenze eines Ausblendungsfensters (T_{blank}) zu bestimmen, um einen Abschnitt des zweiten Verarbeitungssignals von der weiteren Verarbeitung auf Grundlage von mindestens einem Nahfeldereignis (Vs), das von besagter erster Detektionsstufe (162) detektiert wird, auszuschließen.

9. Kabelloses Herzschrittmachergerät (1) nach einem der vorstehenden Ansprüche, wobei der zweite Verarbeitungskanal (17) eine zweite Detektionsstufe (173) zum Detektieren mindestens eines Fernfeldereignisses (As) im zweiten Verarbeitungssignal umfasst.

10. Kabelloses Herzschrittmachergerät (1) nach Anspruch 9, wobei die zweite Detektionsstufe (173) dafür konfiguriert ist, besagtes mindestens eine Fernfeldereignis (As) durch mindestens eins von Folgendem zu detektieren:
- Abgleich des zweiten Verarbeitungssignals mit einem Schwellenwert, und Bewertung von mindestens einem morphologischen Merkmal des zweiten Verarbeitungssignals, wobei die Bewertung von mindestens einem morphologischen Merkmal mindestens eins von einer Bestimmung eines Amplitudenwerts aus dem zweiten Verarbeitungssignal umfasst,
- Bestimmung einer Dauer eines Wellenabschnitts des zweiten Verarbeitungssignals,
- Bestimmung einer Anzahl von Schwellenüberschreitungen oder Nulldurchgängen im zweiten Verarbeitungssignal, und
- Bestimmung einer Position eines Wellenabschnitts in einem Detektionsfenster.

11. Kabelloses Herzschrittmachergerät (1) nach Anspruch 9 oder 10, wobei die Verarbeitungsschaltung (15) dafür konfiguriert ist, ein Stimulationssignal auf Grundlage von mindestens einem detektierten Fernfeldereignis (As) zu triggern.

12. Verfahren zum Betrieb eines kabellosen Herzschrittmachergeräts (1) zur Bereitstellung einer intrakardialen Stimulation, umfassend:
Empfangen elektrischer Signale, unter Verwendung einer Elektrodenanordnung, die an einem Gehäuse (10) des kabellosen Herzschrittmachergeräts (1) angeordnet ist; und
Verarbeiten, unter Verwendung einer Verarbeitungsschaltung (15), die im Gehäuse (10) eingeschlossen und mit der Elektrodenanordnung wirkverbunden ist, eines ersten Verarbeitungssignals und eines zweiten Verarbeitungssignals, die aus elektrischen Signalen abgeleitet sind, die über die Elektrodenanordnung empfangen werden, wobei die Verarbeitungsschaltung (15) einen ersten Verarbeitungskanal (16) mit einer ersten Eingangsverstärkung (G1) zum Verarbeiten besagten ersten Verarbeitungssignals, und einen zweiten Verarbeitungskanal (17) mit einer zweiten Eingangsverstärkung (G2) zum Verarbeiten besagten zweiten Verarbeitungssignals umfasst, wobei die zweite Eingangsverstärkung (G2) höher als die erste Eingangsverstärkung (G1) ist;
Unterscheiden, durch eine Verarbeitungsstufe (172), die im zweiten Verarbeitungskanal (17) umfasst ist, eines Wellenabschnitts von einem anderen Wellenabschnitt im zweiten Verarbeitungssignal;
Anwenden, auf das zweite Verarbeitungssignal, durch die Verarbeitungsstufe (172), die im zweiten Verarbeitungskanal (17) umfasst ist, eines Ausblendungsfensters, um einen Abschnitt des zweiten Verarbeitungssignals von der weiteren Verarbeitung auszuschließen; und Verstärkung, durch eine Verstärkungsstufe (170), die im zweiten Verarbeitungskanal (17) umfasst ist, des zweiten Verarbeitungssignals, wobei die Verstärkungsstufe (170) während besagtem Ausblendungsfenster (T_{blank}) abgeschaltet ist.

## Revendications

1. Dispositif de stimulateur cardiaque sans câbles (1) conçu pour pourvoir à une stimulation intracardiaque, le dispositif de stimulateur cardiaque sans câbles (1) comprenant :
un boîtier (10) ;
un agencement d'électrodes agencé sur le boîtier (10) et conçu pour recevoir des signaux électriques ; et
un circuit de traitement (15) renfermé dans le boîtier (10) et relié de manière fonctionnelle à l'agencement d'électrodes, dans lequel le circuit de traitement (15) comprend un premier canal de traitement (16) ayant un premier gain (G1) pour le traitement d'un premier signal de traitement dérivé de signaux électriques reçus au travers de l'agencement d'électrodes et un second canal de traitement (17) ayant un second gain (G2) pour le traitement d'un second signal de traitement dérivé de signaux électriques reçus au travers de l'agencement d'électrodes, le second gain (G2) étant supérieur au premier gain (G1), dans lequel
le second canal de traitement (17) comprend un stade de traitement (172) qui est conçu pour différencier une partie d'onde d'une autre partie d'onde dans le second signal de traitement et appliquer, au second signal de traitement, une fenêtre de masquage pour exclure une partie du second signal de traitement de tout traitement ultérieur ; et dans lequel
le second canal de traitement (17) comprend un stade d'amplification (170) pour amplifier le second signal de traitement, dans lequel le stade d'amplification (170) est désactivé pendant ladite fenêtre de masquage (Tblank).

2. Dispositif de stimulateur cardiaque (1) selon la revendication 1, dans lequel le boîtier (10) comprend une pointe (100), l'agencement d'électrodes comprenant une première électrode (11) placée sur la pointe (100) pour venir en prise avec le tissu intracardiaque (M).

3. Dispositif de stimulateur cardiaque (1) selon la revendication 2, dans lequel l'agencement d'électrodes comprend une deuxième électrode (12) formée par un anneau d'électrode s'étend de manière circonférentielle autour du boîtier (10) et étant placé à une distance de ladite pointe (100).

4. Dispositif de stimulateur cardiaque (1) selon la revendication 3, dans lequel le circuit de traitement (15) est conçu pour traiter, au titre dudit premier et/ou second signal de traitement, un premier et/ou un second signal détecté entre la première électrode (11) et la deuxième électrode (12).

5. Dispositif de stimulateur cardiaque (1) selon au moins l'une des revendications 2 à 4, dans lequel le boîtier (10) comprend une extrémité lointaine (101) opposée à la pointe (100), l'agencement d'électrodes comprenant une troisième électrode (14) agencée dans les environs de l'extrémité lointaine (101).

6. Dispositif de stimulateur cardiaque (1) selon la revendication 5, dans lequel le circuit de traitement (15) est conçu pour traiter, au titre dudit premier et/ou second signal de traitement, un premier et/ou un second signal détecté entre la première électrode (11) et la troisième électrode (13).

7. Dispositif de stimulateur cardiaque (1) selon l'une des revendications précédentes, dans lequel le circuit de traitement (15) est conçu pour traiter le premier signal de traitement pour détecter une activité ventriculaire et le second signal de traitement pour détecter une activité auriculaire.

8. Dispositif de stimulateur cardiaque (1) selon l'une des revendications précédentes, dans lequel le premier canal de traitement (16) comprend un premier stade de détection (162) pour détecter au moins un événement de champ proche (Vs) dans le premier signal de traitement, dans lequel le stade de traitement (172) du second canal de traitement (17) est conçu pour déterminer au moins une limite d'une fenêtre de masquage (T_{blank}) pour exclure une partie du second signal de traitement de tout traitement ultérieur sur la base d'au moins un événement de champ proche (Vs) détecté par ledit premier stade de détection (162).

9. Dispositif de stimulateur cardiaque (1) selon l'une des revendications précédentes, dans lequel le second canal de traitement (17) comprend un second stade de détection (173) pour détecter au moins un événement de champ lointain (As) dans le second signal de traitement.

10. Dispositif de stimulateur cardiaque (1) selon la revendication 9, dans lequel le second stade de détection (173) est conçu pour détecter ledit au moins un événement de champ lointain (As) par au moins une parmi
- la comparaison du second signal de traitement à un seuil, et l'évaluation d'au moins une caractéristique morphologique du second signal de traitement, dans lequel l'évaluation d'au moins une caractéristique morphologique inclut au moins une parmi la détermination d'une valeur d'amplitude du second signal de traitement,
- la détermination d'une durée d'une partie d'onde du second signal de traitement,
- la détermination d'un nombre de dépassements de seuils ou de dépassements de zéro dans le second signal de traitement, et
- la détermination d'un emplacement d'une partie d'onde dans une fenêtre de détection.

11. Dispositif de stimulateur cardiaque (1) selon la revendication 9 ou 10, dans lequel le circuit de traitement (15) est conçu pour déclencher un signal de stimulation à partir d'au moins un événement de champ lointain détecté (As).

12. Procédé de fonctionnement d'un dispositif de stimulateur cardiaque (1) destiné à fournir une stimulation intracardiaque, comprenant :
la réception de signaux électriques, en utilisant un agencement d'électrodes agencé sur un boîtier (10) du dispositif de stimulateur cardiaque (1) ; et
le traitement, en utilisant un circuit de traitement (15) renfermé dans le boîtier (10) et relié de manière fonctionnelle à l'agencement d'électrodes, d'un premier signal de traitement et d'un second signal de traitement dérivés de signaux électriques reçus au travers de l'agencement d'électrodes, dans lequel le circuit de traitement (15) comprend un premier canal de traitement (16) ayant un premier gain (G1) pour le traitement dudit premier signal de traitement et un second canal de traitement (17) ayant un second gain (G2) pour le traitement dudit second signal de traitement, le second gain (G2) étant supérieur au premier gain (G1) ;
la différentiation par un stade de traitement (172) qui est compris par le second canal de traitement (17) d'une partie d'onde d'une autre partie d'onde dans le second signal de traitement ;
l'application, par le stade de traitement (172) qui est compris par le second canal de traitement (17), au second signal de traitement, d'une fenêtre de masquage pour exclure une partie du second signal de traitement de tout traitement ultérieur ; et
l'amplification, par un stade d'amplification (170) qui est compris par le second canal de traitement (17), du second signal de traitement, dans lequel le stade d'amplification (170) est désactivé pendant ladite fenêtre de masquage (Tblank).
